# FASCICULE DE BREVET EUROPEEN

(11) **EP 4 151 741 B1**
(45) Date de publication et mention de la délivrance du brevet: **12.11.2025**
(21) Numéro de dépôt: 22195711.1
(22) Date de dépôt: 14.09.2022
(51) Int. Cl.: C12P 7/649, C12M 1/00, C12M 1/26

(54) **PROCÉDÉ POUR LA SYNTHÈSE ENZYMATIQUE D'UN BIODIÉSEL À PARTIR DE LIPIDES USAGÉS**
VERFAHREN ZUR ENZYMATISCHEN SYNTHESE EINES BIODIESELS AUS GEBRAUCHTEN LIPIDEN
PROCESS FOR THE ENZYMATIC SYNTHESIS OF BIODIESEL FROM USED LIPIDS

(30) Priorité: 14.09.2021 FR 2109639
(43) Date de publication de la demande: 22.03.2023
(73) Titulaire: Gecco SAS, 59710 Avelin (FR); Université de Lille, 59655 Villeneuve-d'Ascq (FR)
(72) Inventeur: HIS, Cédric, 59155 FACHES-THUMESNIL (FR); MILLARES, Michel, 59300 VALENCIENNE (FR); ALJAWISH, Abdulhadi, 59650 VILLENEUVE D ASCQ (FR); FROIDEVAUX, Renato, 59146 Pecquencourt (FR)
(74) Mandataire: AWA Benelux

(56) Documents cités:
- WO-A1-00/12743
- WO-A1-2011/107977
- WO-A1-2012/098114
- WO-A2-2013/116342
- SONARE N R ET AL: "Transesterification of used sunflower oil using immobilized enzyme", JOURNAL OF MOLECULAR CATALYSIS B : ENZYMATIC,, vol. 66, no. 1-2, 1 September 2010 (2010-09-01), pages 142 - 147, XP027145664, ISSN: 1381-1177, [retrieved on 20100428]
- TONGBORIBOON K ET AL: "Mixed lipases for efficient enzymatic synthesis of biodiesel from used palm oil and ethanol in a solvent-free system", JOURNAL OF MOLECULAR CATALYSIS B : ENZYMATIC,, vol. 67, no. 1-2, 1 November 2010 (2010-11-01), pages 52 - 59, XP027289889, ISSN: 1381-1177, [retrieved on 20100915]

## Description

### Objet de l'invention

La présente invention se rapporte au domaine de la synthèse enzymatique et concerne un procédé de synthèse d'un biodiesel composé d'un mélange d'esters éthyliques ou méthyliques, à partir de lipides usagés provenant d'une huile végétale non acide usagée, d'une huile usagée acide et/ou d'une graisse animale usagée.

### Etat de la technique

Actuellement, seuls les esters méthyliques d'huiles végétales (EMHV) et graisses animales sont autorisés en Europe et utilisés comme biocarburant, en particulier comme biodiesel, en les mélangeant avec du diesel à 30% maximum. Néanmoins, ces esters méthyliques d'huiles végétales, comme les EMHV venant d'huiles végétales usagées, doivent répondre à la norme DIN EN14214 qui encadre tous les paramètres physico-chimiques d'un biodiesel, hormis les quantités d'alcool résiduel, uniquement définie pour le méthanol.

Mais la législation évoluant rapidement, les esters éthyliques d'huiles végétales (EEHV) sont en phase d'autorisation par les douanes pour la vente et l'utilisation, si une déclaration est faite dans ce sens. Cependant, la norme DIN EN14214 n'est pas adaptée pour la mesure des quantités d'esters éthyliques d'huiles végétales usagées à cause du standard utilisé.

Le document de brevet WO2012/098114 divulgue un procédé de production d'esters d'alkyle d'acides gras, dans lequel une solution comprenant une matière première comprenant des acides gras, de l'alcool, de l'eau et du glycérol est mise en contact avec une enzyme lipolytique. Dans ce procédé, la matière première comprenant des acides gras peut être une huile ou une graisse usagée.

### Buts de l'invention

La présente invention a pour but d'obtenir un procédé de conception éco-propre, simple, efficace, peu couteux et applicable à partir des sources de lipides disponibles localement pour produire d'un biodiésel à partir des lipides d'une huile végétale usagée normale, d'une huile usagée acide et/ou d'une graisse animale usagée.

De plus, l'invention a pour but d'obtenir un tel procédé qui réduit ou supprime les étapes pour prétraiter chimiquement de la matière première et pour laver le biodiésel obtenu

Un autre but de l'invention est de simplifier les étapes du procédé et de l'appareillage utilisé pour en réduire les coûts.

### Résumé de l'invention

L' invention concerne un procédé d'obtention d'un biodiesel constitué d'un mélange d'esters éthylique ou d'esters méthyliques, par une transformation enzymatique de lipides, choisis parmi le groupe consistant en de l'huile végétale usagée, de l'huile végétale acide et/ou de la graisse animale usagée, dans lequel ladite transformation enzymatique comprend une trans-estérification enzymatique et une hydro-estérification enzymatique, une addition d'alcool, une addition d'eau ou d'une base en solution aqueuse et une addition de glycérol, caractérisé en ce que préalablement à l'addition d'alcool, les lipides, l'eau ou la base et le glycérol forment un milieu homogène et dans lequel ce milieu est mis en contact avec une ou plusieurs enzymes de type triacylglycerol acyl hydrolase.

De préférence, le procédé de l'invention présente aussi une ou plusieurs des caractéristiques suivantes:
L'enzyme est une lipase sélectionnée parmi le groupe consistant en les lipases immobilisées ou non, de type triacylglycérol acyl hydrolases (E.C.3.1.1.3) d'origine bactérienne ou extraits de champignons, telles que les lipases 1,3 spécifiques ou CALB issues de *Pseudomonas cepacia, de Pseudomonas aeruginosa, de Candida Antarctica, de Candida Rugosa, de Rhizomucor miehei, d'Aspergilus niger* ou de *Thermomyces lanuginosus, l'enzyme étant* éventuellement immobilisée sur une résine, en particulier une résine de poly(méthyle)méthacrylate réticulée par du benzène de méthyle et ces enzymes étant identifiées par les noms commerciaux des enzymes Novozym 40116, Novozym 40086, Novozym 435, Lipozyme RM IM, Lipozyme TL IM, CalB Sigma, CalB Immoplus, NovoLime, TL Sigma, Callera TM, Eversa, Pallatase, Eversa Transform 2.0 libre en forme liquide issue de *Thermomyces lanuginosus* ou un mélange de deux ou de plusieurs de ces enzymes.

Les lipides sont soumis, préalablement à la transformation enzymatique en biodiesel, à une ou plusieurs étape(s) de décantation en cascade des lipides, pour en extraire des particules solides présentes dans ces lipides.

Les lipides constitués d'huile de palme usagée ou d'une graisse animale usagée sont chauffés à une température de 40 °C ou plus, de préférence de 45°C ou plus avant la ou les étapes de filtration.

Les lipides sont soumis, préalablement à la transformation enzymatique en biodiesel, à une ou plusieurs étape(s) d'élimination de l'eau, et obtenir des lipides contenant une teneur en eau inférieure à 1% en poids par rapport au poids des lipides.

Les lipides de l'huile végétale usagée et/ou de l'huile végétale acide sont soumis, préalablement à la transformation enzymatique en biodiesel, à une ou plusieurs étape(s) de filtration et de collecte d'une huile végétale ayant une turbidité ou un trouble dans le liquide proche de 0.

Les lipides sont soumis, préalablement à la transformation enzymatique en biodiesel, à une étape de décantation à la température ambiante pour les huiles usagées et à 45°C ou plus pour les graisses animales pendant une durée comprise entre 48 heures et 72 heures.

L'eau ou la base sont additionnés au pourcentage de moins 0,5% et le glycérol est additionné au pourcentage allant de 1% à 10% par rapport aux lipides et dans lequel ce milieu est mise en agitation pendant une durée comprise entre 15 minutes et 1 heure pour former un milieu dit réactionnel et homogène (ou mélange homogène). Puis, ce milieu dit réactionnel (ou mélange) est mis en contact avec l'enzyme pendant une durée comprise entre 30 minutes, de préférence 1 heure et 2 heures pour vérifier l'efficacité enzymatique par hydrolyse et générer des acides gras libre ce qui incite la réaction d'estérification et donc améliore l'efficacité enzymatique et réduit le temps réactionnel. L'efficacité de l'enzyme est réalisée pour identifier les paramètres à modifier dans le processus de synthèse du biodiésel afin d'optimaliser sa production. Cette mesure d'activité est réalisée sur un échantillon avant l'addition d'alcool et avant de déclencher la réaction. Si l'enzyme libère moins de 10% d'acides gras libres après 1 heure, il faut modifier des paramètres comme l'augmentation de la concentration en enzyme, une réduction d'acidité , un mélange plus efficace dans le milieu réactionnel ou une modification de la température ayant un optimum d'activité en fonction de chaque type d'enzyme.

L'alcool est additionné au ratio molaire (alcool : huile) allant de (3:1) à (16:1) de manière séquentielle de 1 à 4 fois, après une durée comprise entre 15 minutes et 4 heures, de préférence après une durée comprise entre 30 minutes et deux heures, pour déclencher la transformation enzymatique des lipides en biodiesel, par addition d''éthanol ou de méthanol.

Le procédé comprend une étape de recyclage de l'enzyme utilisée, du glycérol utilisé et généré ou de l'eau utilisée et générée, avec de préférence une étape de réutilisation de l'enzyme libre avec l'eau et/ou la base et le glycérol.

Le procédé comprend la transformation enzymatique des lipides usagés en biodiesel, une étape de raffinage du milieu réactionnel incluant la séparation de la partie légère (organique) contenant l'alcool, le glycérol et les esters formés lors de la transformation enzymatique, et de la partie lourde (aqueuse) contenant l'enzyme, l'eau et le glycérol du milieu dit réactionnel (mélange) et du biodiesel obtenu.

Le procédé inclut une étape de raffinage qui comprend une évaporation de l'alcool du milieu dit réactionnel (ou mélange), et éventuellement une collecte par condensation de l'alcool évaporé et/ou l'addition de l'alcool recyclé dans le procédé d'obtention du biodiesel selon l'invention.

L'étape du raffinage comprend une décantation du glycérol et/ou de la partie aqueuse du milieu dit réactionnel (ou mélange) .

Une étape de recyclage peut être réalisée de la partie (lourde) aqueuse du milieu dit réactionnel (ou mélange) incorporant l'enzyme est additionnée avec de l'alcool dans le milieu dit réactionnel (ou mélange) contenant les lipides usagés pour l'obtention du biodiesel selon l'invention, sans ajout d'eau de base, de glycérol et d'enzyme.

Le procédé comprend après l'étape de raffinage, une étape de collecte du biodiesel raffiné obtenu, suivi par une étape d'addition d'un antioxydant, par exemple sous forme liquide d'une base phénolique, au biodiesel raffiné et collecté, et/ou une étape de mélange du biodiesel raffiné et collecté avec un autre carburant (diésel)

Il est également divulgué ici (mais ne faisant pas part de l'invention) une installation d'obtention d'un biodiesel par la mise en œuvre du procédé décrit en haut, ladite installation comprenant :
un module de prétraitement de lipides comprenant des moyens de collecte, de décantation en cascade et de filtration de lipides provenant d'une huile végétale usagée non-acide, d'une huile végétale usagée acide et/ou d'une graisse animale usagée, le dit module est de préférence constitué d'un ou plusieurs filtres, de préférence d'une ou de plusieurs grilles filtrantes dont la taille des ouvertures est comprise entre 3 mm et 1 mm, et éventuellement d'un déshuileur, apte à collecter des lipides prétraités, ledit module de prétraitement des lipides étant connecté à
un module réactionnel comprenant un réacteur, un dispositif de chauffe du réacteur, un dispositif d'agitation des réactifs présents dans le réacteur et des moyens d'addition au réacteur des lipides prétraités des moyens d'addition d'eau et/ou d'une base, des moyens d'addition de glycérol et des moyens d'addition d'alcool, ledit réacteur étant connecté à
un module de collecte et de raffinage d'un mélange du biodiesel obtenu et des réactifs présents dans le milieu dit réactionnel (ou mélange) issu du réacteur, ledit module de collecte et de raffinage comprenant des moyens de collecte par décantation du biodiesel raffiné, du glycérol et de la partie aqueuse du milieu dit réactionnel (ou mélange) et des moyens de recyclage du glycérol et de l'enzyme; des moyens d'évaporation du surplus d'alcool et éventuellement des moyens de recyclage de l'alcool présent dans le milieu réactionnel ledit module de collecte et de raffinage étant connecté à
un module de traitement et de collecte du biodiesel raffiné obtenu comportant une cuve et des moyens d'addition à la cuve d'un antioxydant, cette cuve étant connectée à une cuve de mélange du biodiesel raffiné obtenu avec un autre carburant.

La présente invention sera décrite en détails dans les exemples ci-dessous en référence aux figures annexées et présentés à titre d'illustrations d'une forme d'exécution préférée et non limitative de l'invention.

### Brève description des figures

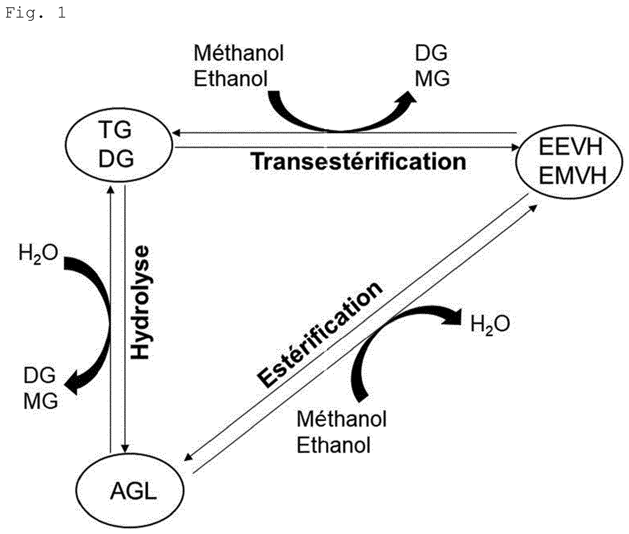

La figure 1 représente les réactions participatives à la transformation des lipides usagés en biodiesel

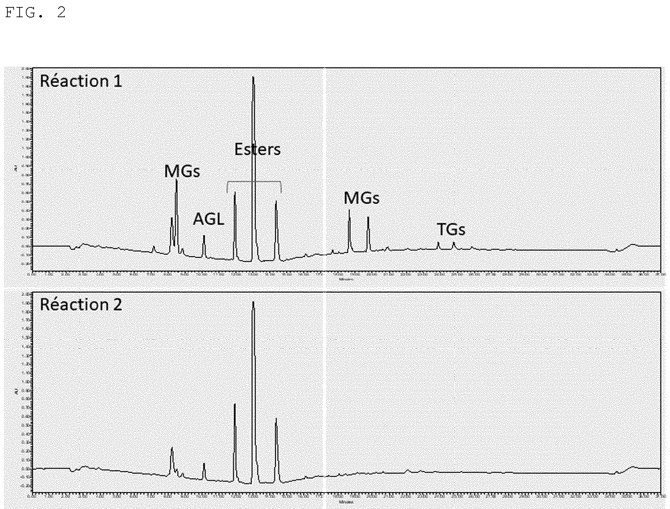

La figure 2 représente une analyse comparative des composés obtenus après deux réactions de synthèse différentes.
La figure 3 représente l'installation

### Description détaillée de l'invention

La présente invention concerne un procédé de synthèse d'un biodiesel, à partir de lipides dits usagés, ce biodiesel étant défini comme un mélange d'esters éthyliques ou méthyliques provenant d'huiles végétales usagées (EEHVU et EMHVU), d'huiles acides et/ou de graisses animales usagées.

Le procédé de l' invention, ainsi que l'installation divulguée ici (mais ne faisant pas part de l'invention), comportent essentiellement plusieurs des modules ou étapes décrits ci-dessous:
A. Un premier module ou une première étape, dit(e) de prétraitement des lipides (formant les matières entrantes à traiter) par décantation en cascade, par des étapes de filtration et par une déshuileur à la température ambiante pour les huiles végétales usagées et à 45°C pour les graisses animales correspondent au module ou à l'étape 1, suivi(e) par
B. un second module ou une seconde étape dit(e) réactionel (le) correspondent au module ou à l'étape 2, cette réaction comprend via des moyens adaptés présents dans le module pour obtenir une trans-estérification (par une alcoolyse) et une hydro-estérification (par hydrolyse et puis par estérification) des lipides, avec des conditions adaptées à chaque type d'huile (huiles végétales usagées, huiles végétales acides et/ou graisses animales), suivi par
C. un troisième module ou une troisième étape dit(e) de récupération de l'enzyme utilisée, du surplus d'alcool, du glycérol dans la seconde étape et puis du biodiesel obtenu, correspondent au module ou à l'étape 3, suivi par
D. un quatrième module ou une quatrième étape dit(e) de collecte et traite du biodiesel (dit « raffiné ») obtenu à la troisième étape est obtenue avec l'addition d'un antioxydant et éventuellement un mélange avec un autre carburant (diesel).

Dans le procédé et l'installation divulgués ici, trois types de lipides dits usagés (utilisées comme matières entrantes) peuvent être traités pour la préparation du biodiésel de l'invention.

Un premier type de lipides est obtenu à partir d'une huile végétale usagée (HVU) qui correspond à une huile issue de la cuisson dans l'huile d'aliments végétaux ou carnés, comme les pommes de terre (frites), les bananes, les pâtes à frire dites pâtes à beignets et à churros ou les viandes, de préférence une huile usagée choisie parmi le groupe consistant en l'huile de palme, l'huile de tournesol et/ou l'huile de colza. Une huile végétale usagée a subi une détérioration due à la cuisson provoquant l'apparition de composés polaires, c'est-à-dire de composés non présents dans l'huile initiale et qui sont donc des produits dégradés et/ou de différents polymères comme de l'amidon, ainsi qu'une augmentation du taux d'acides gras libres, ce taux n'excédant toutefois pas 5 %. De plus, comme la température de cuisson incite la réaction de polymérisation, la viscosité de l'huile végétale augmente en raison des polymères formés, par rapport à une huile végétale non chauffée.

Un deuxième type de lipides est celui d'une huile végétale acide qui est issue de sites industriels ou de stations d'épuration des eaux usées. La caractéristique principale d'une huile acide est qu'elle contient un fort taux d'acides gras libres, ce taux variant de 5 % à 95 % pour une huile végétale acides très dégradée. Leur provenance très hétéroclite en fait un mélange complexe à traiter notamment compte tenu de la présence d'eau, de résidus solides et de composés polluants.

Un troisième type de lipides est obtenu à partir d'une graisse animale usagée qui est issues de la restauration pour faire frire ou cuire des viandes. Une graisse animale usagés présente un profil différent une huile végétale usagée, se distinguant notamment par la présence de stérols d'origine animale comme le cholestérol. De plus, à l'instar de l'huile de palme, une graisse animale est solide à température ambiante, mais le taux d'acides gras libres n'excède toutefois pas les 5 %.

### Le module et l'étape A de prétraitement

Le module ou l'étape A comprend le prétraitement des lipides formant les matières rentrantes du procédé de l'invention. Ce prétraitement permet l'élimination dans les lipides, des résidus ou particules solides, des composés polymériques et de la majeure partie de l'eau résiduelle dans l'huile végétale usagée, l'huile végétale acide et/ou la graisse usagée, pour arriver à un taux d'eau présent dans les lipides compris entre 300 ppm et 1000 ppm.

Dans ce premier module et cette première étape de prétraitement A qui précède le traitement enzymatique, les lipides présents dans une cuve 1 sont traités par une étape de décantation en cascade dans des cuves 2, pour éliminer des lipides traités, les résidus solides en grosse taille qui peuvent boucher les filtres 4 de l'installation et les lipides usagés sont ensuite filtrés à température ambiante. L'huile de palme et les graisses alimentaires usagées étant solides à température ambiante, elles doivent être chauffées à 40°C ou plus pour rester liquides lors de la filtration et avant d'être soumises aux mêmes conditions que celle décrites ci-dessous dans le prétraitement et le processus de traitement enzymatique. La phase de décantation s'effectue, sans additifs, à une température comprise entre 40°C et 50°C de préférence à environ 45°C pendant une durée comprise entre un jour et trois jours et les transferts de matière (par pompage) sont effectués à la même température. L'installation comporte des moyens de chauffe adaptés pour obtenir et conserver cette température.

L'étape de prétraitement par filtration peut être effectuée en laissant passer les huiles usagées ou les graisses animales fondues sur une ou plusieurs filtres, de préférence sur une ou plusieurs grilles filtrantes dont le diamètre des ouvertures ou taille des ouvertures des grilles est le plus grand pour la première grille et diminue avec chaque grille suivante. Une première grille filtrante comporte des ouvertures, comme des trous ayant un diamètre compris entre 2 mm et 4 mm, de préférence d'environ 3 mm. De préférence, une deuxième grille filtrante avec des ouvertures, comme des trous aura un diamètre ou une taille des ouvertures comprise entre 0,5 mm et 2 mm d'environ 1 mm.

Cette filtration préalable permet d'éliminer les particules constituées par les plus gros résidus (alimentaires et non alimentaires), tels que des morceaux de végétaux, de viandes, d'arrêtes de poissons ou de morceaux de papier.

Ensuite, l'huile obtenue passe de préférence par un déshuileur 3, qui joue un rôle inverse et permet l'élimination de l'eau et un débourbage des matières grasses de lipides traités. Lors de cette opération, l'huile traverse le déshuileur à plusieurs compartiments, selon un débit de préférence compris entre 2 m3/heure et 5 m3/heure. L'eau est régulièrement éliminée soit par des vannes manuelles, soit par une vanne automatique qui détecte le trop-plein d'eau. La teneur réduite en eau, suite à cette opération, de l'huile traitée est de préférence inférieure à 1 %.

L'huile obtenue réduite en eau ou la graisse animale est acheminée, de préférence via plusieurs filtres 4, de préférence des grilles filtrantes présentant des ouvertures de 100 µm à 5 µm, vers une cuve de stockage 5 afin de recueillir une graisse animale ou une huile réduite en eau ayant une turbidité proche de 0. La turbidité est la mesure du trouble dans un liquide mesurée par un turbidimètre InFit de Metler Toledo.

### Le module ou l'étape B réactionnel(le)

Dans le procédé et l'installation divulgués ici, les lipides usagés présents dans la cuve de stockage 22 sont soumis à la première décantation avant d'être additionnés à un réacteur 21 pour y subir une transformation enzymatique. Le réacteur 21 comporte des moyens (23, 24, 25, 26 & 27) d'addition de différents réactifs (lipides usagés, enzymes, eau, base, alcool, glycérol, ...) utiles à la réaction et de collecte des produits obtenus ou des produits recyclés (alcool, glycérol, biocarburant, enzymes, ...) vers des cuves ou modules (31, 32, 33, 34, 35, 37 & 37) de traitements.

### A.1.Conditions opératoires pour le traitement d'une huile végétale usagée non acide

La transformation d'une huile végétale usagée non acide en biodiesel consiste à hydrolyser-estérifier/trans-estérifier par réaction enzymatique les différents glycérides (triglycérides, diglycérides et monoglycérides) présents dans l'huile végétale non acide usagée, via une catalyse enzymatique. Dans le procédé de l'invention, l'efficacité de l'enzyme a été augmentée en préparant un milieu homogène entre l'huile usagée et l'eau ajoutée et puis en réagissant l'enzyme avec l'huile usagée pour générer des acides gras libres et inciter la réaction d'estérification, avant d'ajouter l'alcool et de déclencher la transestérification, car la vitesse de la réaction d'estérification est plus rapide que celle de la réaction de trans-estérification. Ces deux étapes ont amélioré l'efficacité l'enzyme ce qui a permis de réduire la quantité d'enzyme nécessaire et le temps réactionnel pour terminer la réaction. L'efficacité de l'enzyme est aussi améliorée en ajoutant une base à faible concentration ce qui a pu réduire la quantité nécessaire de l'enzyme pour réaliser ce procédé et donc le temps réactionnel. L'addition de glycérol permet de réduire la quantité d'eau nécessaire pour générer l'activité enzymatique et donc réduire l'acidité finale (de près de 30%) dans le biodiesel obtenu. De plus, l'enzyme préférée dans ce procédé est présente sous forme liquide, n'est pas couteuse et a pu avantageusement être réutilisée au moins deux fois et ne pose pas de problèmes liés au type d'agitation mécanique.

La réaction enzymatique a été optimisée avec l'addition d'éthanol ou du méthanol).

### A.2.Addition de l'enzyme

Afin de réaliser la transformation enzymatique de l'huile végétale usagée en biodiesel, les enzymes utilisées sont des lipases, de leur nom scientifique triacylglycérol acyl hydrolase (E.C.3.1.1.3) d'origine bactérienne ou extraits de champignons.

Plusieurs lipases commerciales ont été testées et la réaction s'est avérée efficace avec plusieurs d'entre elles comme les lipases issues des souches *Thermomyces lanuginosus, Pseudomonas cepacia, Pseudomonas aeruginosa, Candida Antarctica, Candida Rugosa, Rhizomucor miehei* ou encore *Aspergilus niger.*

Les enzymes peuvent être utilisées dans trois différents états : immobilisées, non immobilisées ou lyophilisées. L'immobilisation de l'enzyme sur un support solide permet de récupérer plus facilement de l'enzyme et d'améliorer la stabilité et la résistance thermique, mais augmente généralement le coût de l'enzyme utilisée, partiellement compensé par une éventuelle réutilisation de l'enzyme, ainsi que la possibilité d'abimer le support de ces enzymes par agitation mécanique.

Par contre, si les enzymes libres sont peu couteuses, leur récupération est difficile et leur stabilité faible, il est cependant possible d'utiliser à nouveau dans le même réacteur ladite enzyme présente dans la portion aqueuse résiduelle de la réaction.

Les trois états ont été testés pour différentes enzymes. En particulier, les trois états ont été testés pour des enzymes issues de Thermomyces lanuginosus, de Candida rugosa, de Candida antartica, et de Rhizmucor miehei.

Certains essais ont aussi été réalisés avec des enzymes non immobilisées notamment de l'Eversa, Callera TM Trans L, Novozym 40116, Lipozyme TL IM ou de la Pallatase. Certains essais ont aussi été réalisés avec des enzymes lyophilisées (en particulier l'enzyme issue de Pseudomonas cepacian et Porcine pancreas). Parmi les enzymes qui ont montré une haute efficacité de la transformation d'une huile usagée normale ou acide et d'une graisse animale en biodiesel, l'enzyme commerciale « Eversa Transform 2.0 » a réalisé le meilleur rendement. En utilisant cette enzyme, le pourcentage d'esters dans le biodiesel a atteint à plus de 96%. Eversa Transform 2.0 utilisée est libre en forme liquide issue de Thermomyces lanuginosus.

La quantité d'enzyme a une influence très importante sur l'efficacité de la réaction. Si l'on en met trop peu, la réaction trop lente ne se déroule pas sur une échelle de temps rentable. Si l'on met une trop grande quantité d'enzyme, l'enzyme se sera auto-inhibée en captant trop de réactifs sans poursuivre la réaction.

Avantageusement, une quantité optimale combinée avec l'intégralité des autres paramètres, permet de faire la réaction sur une durée de moins de 24 heures.

La plage de test pour laquelle les expériences ont été faisables s'étend de (environ) 1 % (v/v) à (environ) 10 % (v/v) pour les enzymes libres, de 0,5 % (p/v) à 5% (p/v) pour les enzymes immobilisées et lyophilisées. En optimisant, la quantité d'eau, la quantité d'alcool, l'ajout d'additif (carbonate de sodium ou hydroxyde de sodium), l'ajout de glycérol et la méthode de préparation du milieu réactionnel avant d'ajouter l'enzyme... on peut réduire la quantité d'enzyme ajoutée à 1% d'enzyme libre et 0,5% d'enzyme immobilisée avec un rendement obtenu environ de 97% d'esters dans le biodiesel.

### A.3.Addition de l'alcool

L'alcool sert de réactif pour l'hydro-estérification/la trans-estérification (accepteur d'acyl) et aussi de solvant pour améliorer la solubilité de l'huile et donc réduire sa viscosité.

La réaction entre l'huile et l'alcool en présence de l'enzyme est expliqué par le schéma suivant :

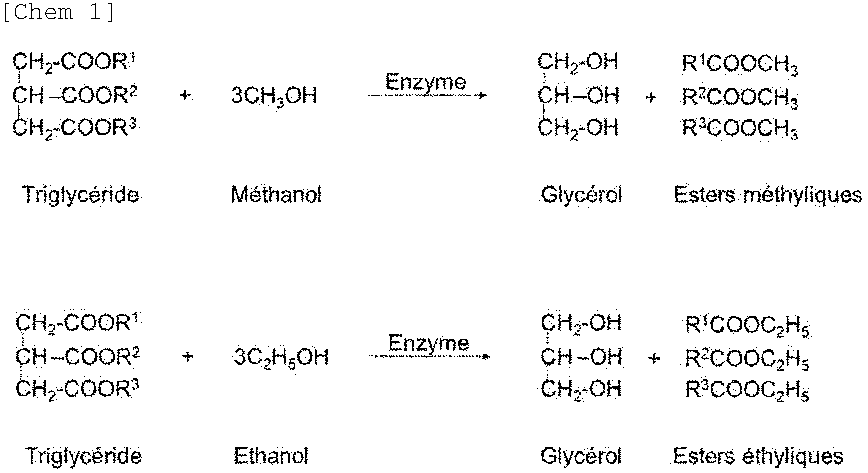

L'addition de plusieurs alcools à courte chaine carbonée (le méthanol ou l'éthanol ) a été testé.

L'éthanol est choisi pour obtenir une transformation optimale des huiles en biodiesel, car cet alcool est peu toxique et comme le méthanol peut-être obtenu via un processus de traitement d'une matière biologique renouvelable par exemple via une fermentation de biomasse. Même si le biodiesel issu des esters éthyliques n'est pas encore autorisé comme le biodiesel issu des esters méthyliques, le procédé a été aussi testé en présence d'éthanol, car l'éthanol peut être aussi obtenu, via un processus de traitement d'une matière biologique renouvelable.

Différents ratios (alcool/huile) de (environ) 3 : 1 (mol/mol) à (environ) 16 : 1 (mol/mol) ont été testés. Comme le triglycéride contient trois acides gras, la quantité d'alcool doit être trois fois plus élevée que celle de triglycéride. Donc, le ratio molaire (3 :1) représente le ratio minimal pour réaliser cette opération. L'excès de l'alcool est indispensable pour orienter la réaction vers la synthèse des esters à condition que cet excès ne provoque pas l'inhibition de l'enzyme. Dans cette étude, le ratio molaire (16 :1) représente le ratio maximal qui ne fait aucune inhibition enzymatique. En conséquence, la plage de ratio molaire allant de (3 :1) au (16 :1) a achevé un pourcentage d'esters dans le biodiesel entre 80% et 97%.

A l'instar d'autres réactifs chimiques, certains alcools peuvent déformer la conformation tridimensionnelle normale des protéines enzymatiques, qui vont perdre leur activité. Cet effet dépend de la nature et l'état de l'enzyme. Pour éviter cet effet, l'alcool a été de préférence ajouté de manière séquentielle pendant la réaction, de préférence avant d'ajouter l'enzyme, pour réduire le contact direct entre une concentration élevée de l'alcool et l'enzyme. Pour cela, l'ajout d'alcool a été testé d'une seule addition à quatre additions ou plus après différents intervalles de temps allant d'environ 30 minutes à environ 2 ou 3 heures. En effet, jusqu'au ratio molaire (6 :1) (éthanol :huile), l'éthanol est ajouté en seule fois au début de la réaction avant d'ajouter l'enzyme sans aucune inhibition enzymatique alors que l'éthanol est ajouté en plusieurs fois à partir du ration molaire (7 :1) pour éviter l'inhibition enzymatique, car à ce ratio, l'enzyme a perdu environ 15% de son activité, quand l'éthanol est ajouté en seule fois. Pour le méthanol, l'ajout est réalisé en plusieurs fois à partir du ratio molaire (3 :1), car le méthanol est plus inhibant que l'éthanol pour l'enzyme, sa chaine de carbone étant plus courte, il peut bloquer le site actif d'enzyme.

### A.4.Addition d'eau

L'eau présente assure la stabilité de l'activité d'enzyme et du déroulement de la réaction, car la couche aqueuse autour de l'enzyme aide à maintenir la conformation tridimensionnelle normale des protéines enzymatiques. L'activité de l'eau est propre à chaque enzyme (type, nature). Ici, à l'instar d'autres paramètres, la quantité d'eau a été optimisée pour réaliser la meilleure activité enzymatique et le meilleur rendement. Même sans eau, la réaction a lieu, mais elle présente une cinétique extrêmement lente. Une grande quantité d'eau amène la réaction vers une hydrolyse réduisant, à son tour, la synthèse des esters et donc le rendement final d'obtention du biodiesel. Au-delà d'une certaine quantité d'eau, la réaction n'a plus lieu, l'eau inhibant la réaction de synthèse et l'amenant 100 % vers une hydrolyse. La réaction optimale a lieu pour des teneurs en eau comprises entre 0% (v/v) sans eau (mais sans eau avec de préférence un ajout de glycérol) avec un pourcentage d'esters d'environ 65% en bénéficiant de l'eau générée par la réaction d'estérification et 5 % (v/v) d'eau avec un pourcentage d'esters d'environ 55% à cause de l'hydrolyse incitée par la quantité élevée d'eau dans le milieu réactionnel. Entre 0% et 5% d'eau, un pourcentage maximal d'esters a atteint à 97%. Pour éviter l'hydrolyse provoquée par l'eau ajoutée et l'eau générée, du glycérol est ajouté en début de traitement, de préférence avant l'addition de l'enzyme dans le milieu réactionnel pour réduire la quantité d'eau ajoutée et créer des microgouttelettes hydrophiles où l'enzyme travaille. Le pourcentage de glycérol allant de 1% à 20% est testé en absence et en présence de l'eau à faible quantité. En général, l'ajout de glycérol améliore le rendement pour l'enzyme libre en réduisant la possibilité de l'hydrolyse notamment aux concentrations modérées vers 5%. De plus, l'ajout d'une base à faible concentration allant de 1% à 4% au lieu de l'eau a pu améliorer l'activité enzymatique et donc réduire la quantité d'enzyme d'environ 25% à 40% et le temps réactionnel d'environ 8h en comparaison avec la réaction sans base ajoutée, car le catalyseur enzymatique présente une meilleure activité en milieu basique (à une valeur supérieure à 7, de préférence à un pH de 8).

### A.5.Modification de la température

L'augmentation de la température améliore la réaction et permettra d'estimer le coût de la production du biodiesel obtenu. Une température très élevée entraine une dénaturation thermique irréversible de la protéine enzymatique, déformant la conformation tridimensionnelle normale de la protéine enzymatique. Même avec une température au-dessous de la température optimale, la réaction a lieu mais a des cinétiques extrêmement lentes. La température optimale est spécifique à chaque enzyme et est indépendante des choix du manipulateur. Dans le cas, de l'enzyme Novozym 40086, la température optimale de réaction est à 30 °C. Certaines enzymes possèdent une tolérance plus forte aux variations de température comme la Novozym 435, dont la température optimale est située à 40 °C mais qui possède tout de même une forte activité entre (environ) 30 °C et (environ) 60 °C. En général, la température de la réaction utilisée dans le procédé de l'invention est comprise entre 30 °C et 70 °C, de préférence entre 35 °C et 55 °C, de préférence entre 40 °C et 50 °C.

### A.6.Agitation dans le milieu réactionnel

Ce paramètre opérationnel assure la répartition équitable des enzymes et des réactifs de façon homogène dans le milieu réactionnel et former une émulsion homogène qui facilite la fonction de l'enzyme qui agit à l'interface d'un milieu organique/aqueux (ici huile/alcool). En effet, les enzymes adoptent une forme ouverte active au passage du milieu aqueux au milieu organique. Une faible agitation entraine une mauvaise répartition des enzymes et des réactifs ce qui ralentit la vitesse de réaction et donc allonge le temps nécessaire pour finir la réaction, ainsi qu'une inhibition de l'enzyme à certains endroits où l'alcool est en forte concentration. Une forte agitation peut déformer les enzymes notamment immobilisées en détériorant le support ou en détachant l'enzyme du support rendant l'enzyme inutilisable et irrécupérable. Pour cela, la réaction a lieu pour des vitesses d'agitation comprise entre 200 tours/minute et 1000 tours/minute, de préférence entre 300 tours/minute et 900 tours/minute. Dans cette gamme, le pourcentage d'esters dans le biodiesel s'est trouvé entre 70% et 97% et le meilleur rendement a été trouvé aux agitations modérées.

La forme du réacteur (21) du module B de l'invention, est celle d'un cylindrique à fond conique et à double paroi jacket avec une pale en forme d'hélice ou une autre forme. Le double paroi du réacteur (21) va assurer une température stable et optimale durant la réaction. De plus, la pale en forme d'hélice va assurer l'agitation équitable du contenu du milieu de réaction pour obtenir une émulsion homogène. A l'échelle de laboratoire, l'optimisation des paramètres opérationnels a été réalisée dans des réacteurs batch en verre avec un volume allant entre 100 ml et 10 litres. A l'échelle d'un pilote, une réaction optimale a été testée au volume de 500 litres dans un réacteur à paroi métallique. A l'échelle industrielle, la production du biodiesel a été effectuée dans un réacteur à paroi métallique au volume de 5000 litres.

### A.7.Préparation du milieu réactionnel

Comme l'enzyme (lipase) agit à l'interface d'un milieu organique/aqueux, une préparation pour former une émulsion homogène avant d'ajouter l'enzyme est réalisée, ce qui facilite l'activité efficace de l'enzyme et assure une température optimale et homogène partout dans le milieu réactionnel. Avant d'ajouter l'enzyme, un mélange huile végétale et eau (ou avec une base) est agité pendant une durée comprise entre 15 minutes et 2 heures, de préférence entre 30 minutes et 1 heure.

Pour augmenter la surface d'échange entre l'enzyme (phase aqueuse) et les triglycérides (phase organique) et pour réduire la possibilité d'hydrolyse par l'eau, du glycérol est ajouté. Ensuite, la lipase est ajoutée dans le milieu réactionnel pendant un certain temps pour s'adapter avec le milieu avant d'ajouter l'alcool et en même temps pour hydrolyser une certaine quantité de triglycérides ce qui accélère la vitesse réactionnelle en amenant la réaction d'estérification qui est plus rapide que la réaction de trans-estérification. Cette étape améliore l'efficacité de l'enzyme qui augmente à son tour, la réaction et réduit le temps nécessaire pour transformer les triglycérides des lipides en biodiesel. Enfin, l'alcool est ajouté en plusieurs fois pour déclencher la trans-estérification des triglycérides.

### B. Conditions opératoires pour le traitement d'une huile végétale acide

Une huile végétale peut présenter un fort taux d'acide gras libres. L'objectif est de valoriser l'intégralité des glycérides et acides gras présents dans l'huile végétale acide usagée via deux réactions : une estérification et une trans-estérification, toutes les deux catalysées par des enzymes. L'estérification est une réaction permettant de transformer les acides en esters en les faisant se condenser avec un alcool.

Trois méthodes peuvent être utilisées pour faire cette réaction. Soit la réaction se passe en deux étapes. Durant la première étape, l'huile végétale acide usagée ayant subie le prétraitement est mise en réaction avec de l'éthanol ou du méthanol et une première enzyme pour estérifier les acides gras libres en esters éthyliques ou méthylique. Ce mélange est ensuite récupéré, puis mis en réaction avec une deuxième enzyme afin de trans-estérifier les glycérides restantes. Tous les composés de l'huile végétale acide usagée sont ainsi devenus des esters éthyliques ou méthylique. L'estérification, contrairement à la trans-estérification, générera de l'eau.

Une deuxième méthode consiste à faire réagir le mélange éthanol ou méthanol/huile végétale acide avec une seule enzyme qui effectue l'estérification et la trans-estérification.

Une troisième méthode consiste à transformer les acides dans l'huile végétale acide usagée en triglycérides par l'estérification avec le glycérol à température élevée et puis la transformation de ces triglycérides en biodiesel en présence de l'éthanol ou du méthanol par catalyse enzymatique.

Plusieurs enzymes commerciales immobilisées comme Lipozyme TL IM, Novozym 40086, Lipozyme RM IM, CalB Sigma, CalB Immoplus, NovoLime, Novozym 435, ainsi que des enzymes commerciales libre en forme liquide comme TL Sigma, Callera TM, Eversa, Novozym 40116, RM liquide, Pallatase de *Candida rugosa* ont été testées.

Ces trois méthodes dépendent principalement de la nature de l'enzyme. Certaines enzymes sont 1,3-spécifique, 3 spécifique ou ne possèdent aucune spécificité. De la même façon, certaines enzymes sont plus affines pour l'estérification, la trans-estérification ou les deux.

Par exemple, la Novozym 435 ne possède pas de spécificité et peux donc réagir avec les acides gras libres et les glycérides et donc faire l'estérification et la trans-estérification en même temps. Cela modifie cependant la cinétique réactionnelle de chaque réaction.

La Lipozyme RM IM est une enzyme très efficace en estérification. La Lipozyme TL IM est très efficace en trans-estérification et Il est possible de faire réagir huile acide et éthanol tout d'abord avec la Lipozyme RM IM, puis avec la Lipozyme TL IM.

L'enzyme immobilisée Novozym 435 peut transformer l'huile acide à 75% d'acides libres en biodiesel contenant 95% d'esters éthyliques, après 24 heures en présence de l'éthanol comme accepteur d'acyle.

### C. Conditions opératoires pour le traitement d'une graisse animale usagée (gras de bœuf)

Pour le traitement d'une graisse animale usagée, à l'instar d'une huile végétale, une réaction enzymatique est optimisée.

Deux méthodes de trans-estérification ont été utilisées :
la trans-estérification dans un solvant organique : la graisse animale usagée est mise en réaction avec l'éthanol ou le méthanol et une enzyme commerciale en présence d'hexane permettant une meilleure dissolution des graisses.
la trans-estérification sans solvant organique : la graisse animale usagée est mise en réaction directement avec l'éthanol ou le méthanol et une enzyme commerciale à température qui permet de garder la graisse en forme liquide. Cette méthode demande une agitation beaucoup plus forte, surtout en début de réaction et présente une cinétique plus lente. Une deuxième façon d'optimiser la cinétique de cette réaction est de faire la réaction à plus haute température (comprise entre environ 40 °C et environ 50 °C) avec des enzymes adaptées ou résistantes à ces températures. Par exemple, l'activité de Lipozyme TL IM est fortement diminuée au-delà de 35 °C. A l'inverse, certaines enzymes comme la Novozym 40086 ont des plages d'activité par rapport à la température réactionnelle beaucoup plus importantes.

Trois types d'enzymes ont été testées : immobilisées (TL-IM, Novozyme 40086, Lipozyme RM IM, CalB Sigma, CalB Immoplus, NovoLime, Novozym 435), libres en solution comme TL Sigma, Callera TM, Eversa, Novozym 40116, RM liquide, Lipozyme TL IM, Pallatase, Candida rugosa) et lyophilisées (*Candida rugosa*)*.*

L'enzyme libre Novozym 40116 a pu transformer une graisse de bœuf en biodiesel à 80% d'esters éthyliques à 40°C après 24 heures.

### Le module ou l'étape C de recyclage des enzymes et de raffinage du biodiesel obtenu

Avantageusement et afin d'améliorer en termes économique et écologique, la production du biodiesel, les enzymes utilisées immobilisées peuvent être recueillies du milieu réactionnel et nettoyées afin de les recycler et les réutiliser, de préférence dans le procédé de l'invention. Pour cela, l'enzyme est lavée en circuit fermé dans le module C avec de l'hexane absolu et est puis séchée pendant une nuit à température ambiante. En utilisant cette méthode de lavage, l'enzyme a été réutilisée plusieurs fois en gardant presque 95% de son activité. Ce module et cette étape 3 n'est utile que pour les enzymes immobilisées. Par exemple, en appliquant cette méthode de recyclage, Novozym 435 a été réutilisée 10 fois pendant une durée d'un mois et en gardant presque 95% de son activité initiale.

Pour les enzymes libres présente en forme liquide, le recyclage de l'enzyme est complexe, car il est difficile à récupérer du milieu réactionnel. Cependant, l'enzyme Eversa a été pu être réutilisé au moins deux fois en la laissant dans le milieu réactionnel et en ajoutant de nouveau des réactifs (huiles et alcool) sans addition d'eau.

Pour éviter de générer des déchets et de perdre des réactifs potentiels ou sous-produits valorisables, le procédé de raffinage du biodiesel brut obtenu se fait en deux sous-étapes : une étape d'évaporation de l'alcool résiduel dans le mélange et une étape de décantation naturelle du glycérol et de l'eau générée du biodiesel obtenu durant la réaction.

L'étape d'évaporation a été optimisée afin d'éliminer la majorité, de préférence l'intégralité du surplus d'alcool et récupérer ce surplus d'alcool pour le remettre en réaction dans le procédé de l'invention.

L'évaporation se fait dans le module C, c'est-à-dire une cuve (34) maintenue sous une pression résiduelle d'environ 150 mbar et une température de chauffe du mélange d'environ 120 °C. Ces conditions ne sont que peu modulables, sauf à travailler sous des pressions résiduelles plus faibles allant jusqu'à la valeur d'environ 30 mbar, permettant ainsi de réduire le temps d'évaporation utilisant un évaporateur à flot tombant.

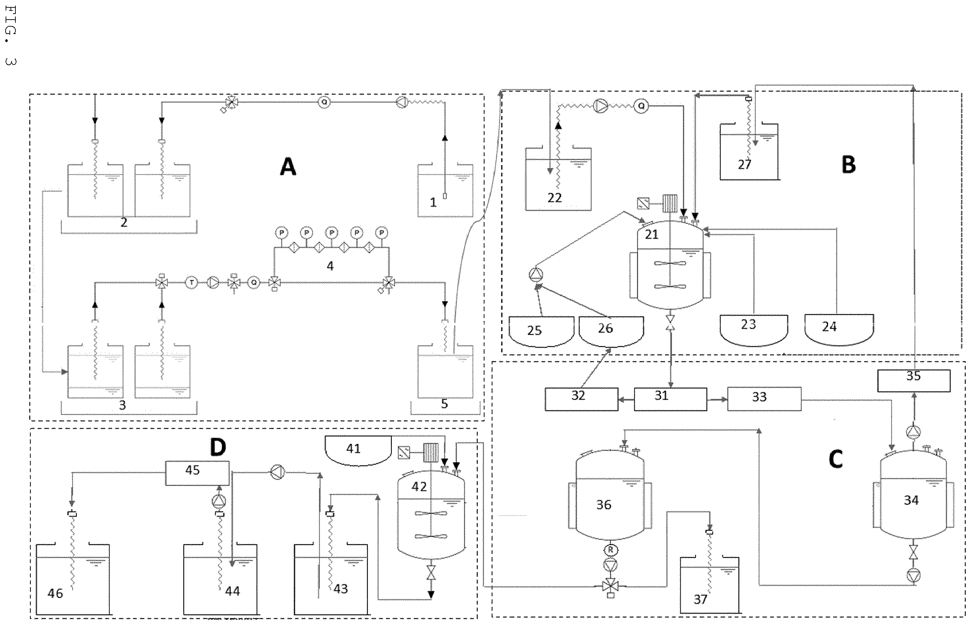

Le liquide arrive dans la partie supérieure de la colonne d'évaporation et coule le long des tubes chauffés. L'appareil est mis en dépression (à environ 150 mbar) et l'alcool s'évapore alors pour être condensé dans un condenseur (35). L'alcool recueilli (pur à 99 %) est ensuite réutilisé dans la réaction de trans-estérification (voir figure 3).

Après avoir évaporé tout l'alcool (35) , le mélange est transféré dans une cuve (36) de décantation du module où le glycérol (37) et le biodiesel se séparent naturellement. Trois phases sont récupérées : le glycérol, un mélange de glycérol et de biodiesel raffiné obtenu constitué de biodiesel pur et le biodiesel pur (B100). Les deux premiers produits (glycérol et glycérol/biodiesel) sont conservés dans des autres cuves de 1000 litres.

Le glycérol obtenu peut être valorisé sur les marchés de la chimie (production de savon industriel) voire de l'agro-alimentaire ou de la cosmétique, le mélange glycérol-biodiesel étant réintroduit dans le module de raffinage. Le B100 est, quant à lui, transféré, via une pompe, dans le module D de traitement du biodiesel décrit ci-dessous.

### Le module ou l'étape D de traitement du biodiesel raffiné

Le module D de traitement du biodiesel raffiné obtenu du module ou étape comporte une cuve (42), où un antioxydant (41 est additionné au biodiesel raffiné obtenu à hauteur de 0,8% et ce biodiesel raffiné obtenu après traitement par lavage à l'eau (43) et purification sur résine (44), peut ensuite être transféré dans une autre cuve (36) et additionné de diesel à hauteur de 70 % afin d'obtenir un biodiesel B30.

L'avantage le plus important du procédé et de l'installation divulgués ici est la possibilité d'utiliser le même catalyseur enzymatique pour déclencher la transformation des huiles usagées dites normales ou acides et des graisses animales en biodiesel. Il s'agit d'un procédé dit « éco-propre », car à l'inverse des procédés chimiques utilisant des catalyseurs acides ou basiques, un tel procédé ne donnera que le biodiesel (comme produit principal) et le glycérol (comme coproduit), sans réactions secondaires (processus de saponification), grâce à la spécificité et la sélectivité de l'enzyme.

Dans le procédé de l'invention, l'efficacité de l'enzyme utilisée est aussi augmentée en préparant un milieu homogène entre l'huile usagée et l'eau ajoutée et puis en réagissant l'enzyme avec l'huile usagée avant d'ajouter l'alcool et de déclencher la trans-estérification. Ces deux étapes ont donc permis de réduire avantageusement la quantité d'enzyme nécessaire pour effectuer la réaction.

L'efficacité de l'enzyme a pu aussi être améliorée en ajoutant une base (une solution de NaOH ou de Na₂CO₃ )à faible concentration et/ou du glycérol ce qui peut contrôler l'acidité dans le biodiesel et réduire la quantité nécessaire de l'enzyme pour réaliser ce procédé et donc le temps réactionnel. De plus, l'enzyme la plus efficace dans ce procédé est présente sous forme liquide, n'est pas couteuse et a pu être réutilisée au moins deux fois, sans perdre aucune activité et sans poser de problèmes liés au type d'agitation mécanique, contrairement aux enzymes immobilisées.

Ce procédé peut être avantageusement combiné à une collecte locale des lipides des huiles usagées et des graisses animales usagées et à l'utilisation locale de ce biodiesel pour une circulation de véhicules.

### Exemples

### Exemple 1: Transformation d'un échantillon d'une huile végétale en biodiesel

100 ml d'une huile végétale obtenue est versée dans un réacteur 20 de 400 ml et puis 3 % (v/v) d'eau ou de base ainsi que du glycérol (5%) sont mélangés avec l'huile pendant 30 minutes, puis 2 % (v/v) d'uneenzyme lipase de type triacylglycérol acyl hydrolases (Eversa Transform 2.0 sous forme liquide) est ajoutée dans le milieu réactionnel (ou mélange) qui est laissé pendant 1 heure sans alcool et donc sans déclencher la réaction de trans-estérification. Cette étape fait réagir l'enzyme avec l'huile pour générer des acides gras et afin d'accélérer la réaction. Ensuite, de l'éthanol au ratio molaire (4:1) par rapport à l'huile végétale est ajouté en trois fois à des intervalles de temps de 0, 2 et 4 heures. Après environ 22 heures, la séparation de la phase organique et de la phase aqueuse a été réalisée par centrifugation à 4700 rpm pendant 15 minutes.

Pour la production du biodiesel issu des esters méthyliques, le même procédé pour les esters éthyliques comme décrit au-dessus a été appliqué, sauf que le ratio molaire (Méthanol : huile) est de 6 :1.

Le contenu du biodiesel raffiné obtenu a été étudié par des méthodes chromatographiques (CL-HP et CPG).

### Exemple 2: Transformation d'un échantillon d'une huile végétale acide en biodiesel

Dans la présente invention, la transformation des huiles végétales acides en biodiesel a été catalysée par la même enzyme qui a été utilisée dans l'exemple 1, pour la transformation des huiles végétales usagées normales en biodiesel. Pendant cette réaction, l'étape pour bien mélanger de l'huile avec de l'eau ou une base a été supprimé, car la réaction s'est déroulée sans eau ajoutée. La quantité des acides gras libres est très élevée ce qui va inciter la réaction d'estérification en présence de l'alcool. Comme ce dernier type de réaction donne l'ester et de l'eau, la quantité d'eau générée dans le milieu réactionnel va être suffisante pour déclencher la réaction de trans-estérification entre les triglycérides et l'alcool. Ainsi, l'étape pour générer des acides gras entre l'enzyme et l'huile sans alcool a été supprimé, car l'huile utilisée dans ce procédé est déjà acide et contient une quantité élevée des acides gras libre. Le glycérol a été ajouté dans le milieu réactionnel ce qui peut créer un site sur lequel l'enzyme travaille et diminuer l'hydrolyse et donc diminuer la quantité d'acides gras libres.

100 ml d'une huile végétale acide est versée dans un réacteur 20 de 400 ml et 0,5% NaOH et 10% de glycérol ont été ajoutés et puis 1% (v/v) de l'enzyme Eversa ^{®} Transform 2.0 en forme liquide a été ajouté. Enfin, l'éthanol au ratio molaire (4:1) par rapport à l'huile végétale acide a été ajouté en trois fois à des intervalles de temps de 0, 2 et 4 heures pour éviter l'inhibition enzymatique par l'alcool. Après 22 heures, la séparation de la phase organique et de la phase aqueuse a été réalisée par centrifugation à 4700 rpm pendant 15 minutes. Pour étudier le contenu du biodiesel raffiné obtenu, des échantillons ont été analysés par méthodes chromatographiques (CL-HP et CPG). Pour la production du biodiesel issu des esters méthyliques, le même procédé pour les esters éthyliques comme décrit au-dessus a été appliqué, sauf que l'on utilise le ratio molaire (Méthanol : huile) de 6 :1.

### Exemple 3: Transformation d'un échantillon de graisse animale en biodiesel

Dans la présente invention, trois enzymes lipases de type triacylglycérol acyl hydrolases, ont présenté des résultats satisfaits : Lipozyme TL IM, Novozym 40086 et Eversa Tranform 2.0. Donc, la transformation des graisses animales peut être réalisée par le même catalyseur enzymatique (enzyme) utilisé pour transformer les huiles usagées normales ou acides en biodiesel. Comme l'objectif de cette invention est de trouver un procédé éco-propre, la transformation d'une graisse animale en biodiesel a été optimisé sans addition de solvant organique.

100 g de graisses animales a été pesé dans un réacteur 20 de 400 ml et puis 2 % (p/v) d'eau ou de base a été mélangé avec l'huile pendant 30 minutes à la température 45°C pour bien fondre la graisse et bien la mélanger avec de l'eau ou de la base. 4% (v/v) de l'enzyme (Eversa Transform 2.0 sous forme liquide) a ensuite été ajouté dans le milieu réactionnel qui est laissé pendant 1 heure, sans addition d'alcool. Enfin, l'éthanol au ratio molaire (5:1) par rapport aux graisses animales a été ajouté en trois fois à 0, 2 et 4 heures pour éviter l'inhibition enzymatique par l'alcool.

Après 22 heures, la séparation de la phase organique et de la phase aqueuse a été réalisée par centrifugation à 4700 rpm pendant 15 minutes. Pour étudier le contenu du biodiesel raffiné obtenu, des échantillons ont été analysés par méthodes chromatographiques (CL-HP et CPG). Pour la production du biodiesel issu des esters méthyliques, le même procédé pour les esters éthyliques comme décrit au-dessus a été appliqué, sauf que le ratio molaire (Méthanol : graisse) est de 6 : 1.

### Exemple 4:

On mélange (huile usagée + addition d'eau (ou une base) + addition de glycérol) pendant 30 minutes pour obtenir un milieu homogène et éviter le choc thermique et chimique de l'enzyme. Ensuite, la quantité d'enzyme nécessaire est ajoutée pour déclencher l'hydrolyse pendant 1 heure. Finalement, l'alcool est ajouté en plusieurs fois pour éviter l'inhibition enzymatique.

Les inventeurs ont observé que l'étape d'hydrolyse, améliore l'activité enzymatique (la vitesse initiale de l'enzyme avec l'hydrolyse (29,5 mM/h) est presque 2 fois plus importante que celle de l'enzyme sans hydrolyse (16,5 mM/h)).

Cette étape d'hydrolyse améliore la qualité de biodiesel le % en volume des triglycérides et di-glycérides à la fin de réaction suite à l'hydrolyse est proche de zéro, cependant que sans hydrolyse, ces produits représentent de 2-3 % du volume à la fin de réaction),et cette étape d'hydrolyse contrôle le fonctionnement de l'enzyme (il est utile pour tester l'activité enzymatique et savoir si l'activité de cette enzyme fonctionne correctement ou pas, avant d'ajouter l'alcool).

Les inventeurs ont aussi observé que l'addition d'une base, améliore l'activité enzymatique (d'environ 20%) ,ce qui a réduit la quantité d'enzyme nécessaire (de presque 25%) et réduit le temps réactionnel d'environ 8 heures en comparaison de la réaction avec de l'eau (par exemple, avec l'ajout de base, les triglycérides sont totalement transformés après 13h, alors qu'avec l'eau, ils sont transformés après 22 heures).

L'addition d'une base améliore aussi le rendement (c'est-à-dire la quantité de biodiesel fabriqué) d'au moins de 5%: en présence d'une base, l'émulsion (la phase lourde) ne se forme pas, donc il n'y a pas de biodiesel retenu dans le réseau de gel ce qui augmente la quantité de biodiesel obtenu, l'absence de l'émulsion et la présence de deux phases liquides facilitent également la filtration et la récupération du glycérol (coproduit). Par exemple, avec l'addition d'une base, la phase de biodiesel obtenue représente presque 88% du volume, alors qu'avec de l'eau et la formation de l'émulsion, la phase de biodiesel représente autour 82% du volume.

L'addition de la base améliore aussi la qualité de biodiesel (avec l'addition d'une base, les triglycérides et di-glycérides sont presque totalement transformés (100% du volume) et les mono-glycérides sont présent en une quantité minimale (inférieur à 1,5% en volume)).

L'addition de la base réduit aussi l'acidité et la quantité d'acides gras libres restés dans le biodiesel): La présence d'une base a réduit le % d'AGL(par exemple, l'acidité en présence de la base est de presque 2,6%, alors qu'elle est de presque 3% avec de l'eau).

Les inventeurs ont observé que l'ajout du glycérol est utile pour éviter l'hydrolyse provoquée par l'eau ajoutée et l'eau générée, Le glycérol (hydrophile) crée des microgouttelettes hydrophiles favorisant l'activité de l'enzyme.

L'addition du glycérol dans le milieu réactionnel améliore l'acidité : (le % d'acides gras libres non-estérifiés (AGL) est de presque 2,2% en volume en présence du glycérol, comparé à presque 3%. d'AGL en volume avec l'eau).

L'ajout de glycérol améliore aussi le rendement et la réduction d'AGL augmente le rendement d'obtention du biodiesel obtenu d'environ 2% en volume (ar exemple, sans addition de glycérol, le % d'esters obtenu est de presque 90% en volume, alors qu'avec l'addition de glycérol le % d'esters obtenu est de presque 92%).

### Exemple 5:

On réalise une réaction 1 normale sur le mélange (l'huile usagée + 2% eau + 25% alcool + 2,5% enzyme ) et une réaction 2 (avec une hydrolyse + glycérol + addition d'une base) sur de l'huile usagée + 2% base + 4% glycérol + 30 min de mélanger + 2,5% enzyme + hydrolyse 1heure + 25% alcool.

Pour comparer l'efficacité des deux réactions, les inventeurs ont suivi la transformation de triglycérides (TGs) et l'apparition des produits (diglycérides (DGs), monoglycérides (MGs), esters, acides gras libres (AGL)) par chromatographie en phase liquide (HPLC).

Les données sont aussi reprises sur la tableau 1 ci-dessous.

**Tableau 1**

| | Réaction 1 | Réaction 2 |
|---|---|---|
| TGs % | 3, 4 | 0 |
| DGs | 5, 6 | 0 |
| Esters % | 85, 2 | 95, 6 |
| AGL % | 3, 1 | 2, 6 |
| MGs % | 2, 7 | 1, 8 |

La figure 3 est un chromatogramme d'HPLC de la réaction 1 (Huile + eau + enzyme + alcool) et de la réaction 2 (Huile + base + glycérol + mélange 30 minutes + enzyme + hydrolyse 1 heure + alcool).

Dans les chromatogrammes, on constate que la réaction 1 n'est pas terminée, car les triglycérides (TGs) ne sont pas totalement transformés après 24 heures et les produits intermédiaires (di-glycérides, mono-glycérides et les acides gras libres) ne sont pas totalement transformés en esters. Cependant, la réaction 2 est bien terminée et aboutie après 24 heures, car les triglycérides et les produits intermédiaires sont transformés en esters.

## Revendications

1. Procédé d'obtention d'un biodiesel constitué d'un mélange d'esters éthylique ou d'esters méthyliques par transformation enzymatique de lipides choisis parmi le groupe consistant en de l'huile végétale usagée, de l'huile végétale acide et/ou de la graisse animale usagée, dans lequel ladite transformation enzymatique comprend une trans-estérification enzymatique et une hydro-estérification enzymatique, une addition d'alcool, une addition d'eau ou d'une base en solution aqueuse et une addition de glycérol, **caractérisé en ce que** préalablement à l'addition d'alcool, les lipides, l'eau ou la base et le glycérol forment un milieu homogène et dans lequel ce milieu est mis en contact avec une ou plusieurs enzymes de type triacylglycerol acyl hydrolase.

2. Procédé selon la revendication 1, dans lequel le milieu est mis en contact avec l'enzyme pendant une durée comprise entre 30 minutes et 2 heures.

3. Procédé selon la revendication 1 ou 2,dans lequel l'alcool est additionné au ratio molaire (alcool : huile) allant de (3 : 1) à (16 : 1).

4. Procédé selon l'une quelconque des revendications précédentes, dans lequel les lipides sont soumis, préalablement à la transformation enzymatique en biodiesel, à une étape de décantation en cascade et à une ou plusieurs étape(s) de filtration des particules solides présentes dans les lipides sur des filtres et à une étape d'élimination de l'eau, ainsi obtenant des lipides contenant une teneur en eau inférieure à 1% en poids par rapport au poids des lipides.

5. Procédé selon la revendication 4, dans lequel les filtres présentent des ouvertures comprises entre 100 µm et 5 µm.

6. Procédé selon l'un quelconque des revendications précédentes, dans lequel les lipides sont constitués d'huile de palme usagée ou d'une graisse animale usagée qui sont chauffés à une température de 40 °C ou plus, de préférence de 45°C ou plus, avant la ou les étapes de filtration.

7. Procédé selon l'une quelconque des revendications précédentes 1 à 5, dans lequel les lipides sont soumis, préalablement à la transformation enzymatique en biodiesel, à une étape de décantation à la température ambiante pour les huiles usagées et à 45°C ou plus pour les graisses animales pendant une durée comprise entre 48 heures et 72 heures.

8. Procédé selon l'une quelconque des revendications précédentes, comprenant une étape de recyclage de l'enzyme utilisée.

9. Procédé selon la revendication 8 dans lequel l'étape de recyclage de l'enzyme utilisée comprend une étape de réutilisation de l'enzyme libre sous forme liquide, une étape d'immobilisation de l'enzyme utilisée, une étape de collecte de l'enzyme immobilisée utilisée, une étape de lavage de l'enzyme par de l'hexane et une étape de séchage de l'enzyme collectée.

10. Procédé selon l'une quelconque des revendications précédentes, comprenant après la transformation enzymatique des lipides usagés en biodiesel, une étape de raffinage du milieu incluant une séparation par décantation d'une phase lourde qui contient de glycérol formé lors de la transformation enzymatique, l'eau ou la base en solution aqueuse, et l'enzyme, et d'une phase organique qui contient le biodiesel produit et l'alcool, une séparation de l'alcool et le biodiesel de la phase organique par évaporation de l'alcool, et une collecte par condensation de l'alcool évaporé et/ou une addition de l'alcool recyclé dans le procédé d'obtention du biodiesel, une décantation du glycérol et/ou de la partie aqueuse de la phase lourde, une étape de collecte du biodiesel raffiné, suivie par une étape d'addition d'un antioxydant, , au biodiesel raffiné et collecté, et/ou une étape de mélange du biodiesel raffiné collecté avec un autre carburant pour obtenir un biodiesel final à 30% (B30).

11. Procédé selon la revendication 10, dans lequel une partie aqueuse du milieu incorporant l'enzyme, l'eau ou la base en solution aqueuse et le glycérol formé lors de la transformation enzymatique est additionnée d'alcool et de lipides usagés pour l'obtention du biodiesel, sans ajouter séparément de l'enzyme, de l'eau et/ou de la base et du glycérol.

12. Procédé selon l'une quelconque des revendications précédentes, dans lequel l'alcool est choisi parmi le groupe consistant en de l'éthanol ou du méthanol.

13. Procédé selon l'une quelconque des revendications précédentes, dans lequel l'alcool est additionné audit milieu de manière séquentielle de 1 à 4 fois, après une durée comprise entre 15 minutes et 4 heures.

## Patentansprüche

1. Verfahren zur Herstellung eines Biodiesels, bestehend aus einem Gemisch von Ethylestern oder Methylestern durch enzymatische Umwandlung von Lipiden, ausgewählt aus der Gruppe bestehend aus gebrauchtem Pflanzenöl, saurem Pflanzenöl und/oder gebrauchtem tierischem Fett, wobei die besagte enzymatische Umwandlung eine enzymatische Umesterung und eine enzymatische Hydroveresterung, eine Zugabe von Alkohol, eine Zugabe von Wasser oder einer Base in wässriger Lösung und eine Zugabe von Glycerin, **dadurch gekennzeichnet, dass** vor der Zugabe von Alkohol, die Lipide, das Wasser oder die Base und das Glycerin ein homogenes Medium bilden, in dem dieses Medium mit einem oder mehreren Enzymen des Triacylglycerin-Acylhydrolase-Typs in Kontakt gebracht wird.

2. Verfahren nach Anspruch 1, wobei das Medium für einen Zeitraum zwischen 30 Minuten und 2 Stunden mit dem Enzym in Kontakt gebracht wird.

3. Verfahren nach Anspruch 1 oder 2,wobei der Alkohol in einem molaren Verhältnis (Alkohol: Öl) zwischen (3:1) und (16:1) zugesetzt wird.

4. Verfahren nach irgendeinem der vorhergehenden Ansprüche, wobei die Lipide vor der enzymatischen Umwandlung in Biodiesel einem Dekantierungsschritt in Kaskadenform und einem oder mehreren Schritten der Filtration der in den Lipiden vorhandenen festen Partikel auf Filtern und einem Wasserentfernungsschritt unterzogen werden, wodurch Lipide erhalten werden, die einen Wassergehalt von weniger als 1 Gew.-% bezogen auf das Gewicht der **Lipide enthalten**.

5. Verfahren nach Anspruch 4, wobei die Filter Öffnungen zwischen 100 µm und 5 µm aufweisen.

6. Verfahren nach irgendeinem der vorhergehenden Ansprüche, wobei die Lipide aus gebrauchtem Palmöl oder einem gebrauchten tierischen Fett bestehen, das vor dem bzw. den Filtrationsschritten auf eine Temperatur von 40 °C oder mehr, vorzugsweise 45 °C oder mehr, erhitzt wird.

7. Verfahren nach irgendeinem der vorhergehenden Ansprüche 1 bis 5, wobei die Lipide vor der enzymatischen Umwandlung in Biodiesel einem Dekantierungsschritt bei Raumtemperatur für gebrauchte Öle und bei 45 °C oder mehr für tierische Fette für einen Zeitraum zwischen 48 Stunden und 72 Stunden unterzogen werden.

8. Verfahren nach irgendeinem der vorhergehenden Ansprüche, umfassend einen Schritt zum Recycling des gebrauchten Enzyms.

9. Verfahren nach Anspruch 8, wobei der Recyclingschritt des verwendeten Enzyms einen Schritt zur Wiederverwendung des freien Enzyms in flüssiger Form, einen Schritt zum Immobilisieren des verwendeten Enzyms, einen Schritt zum Sammeln des verwendeten immobilisierten Enzyms, einen Schritt zum Waschen des Enzyms mit Hexan und einen Schritt zum Trocknen des gesammelten Enzyms umfasst.

10. Verfahren nach irgendeinem der vorhergehenden Ansprüche, umfassend nach der enzymatischen Umwandlung der verwendeten Lipide in Biodiesel einen Raffinationsschritt des Mediums, der eine Trennung durch Dekantieren einer schweren Phase, die das bei der enzymatischen Umwandlung gebildete Glycerin, Wasser oder Base in wässrige Lösung enthält, und das Enzym umfasst, und einer organischen Phase, die den hergestellten Biodiesel und den Alkohol enthält, eine Trennung von Alkohol und Biodiesel aus der organischen Phase durch Verdampfung von Alkohol und eine Sammlung durch Kondensation von verdampftem Alkohol und/oder Zugabe von recyceltem Alkohol im Prozess der Biodieselherstellung, ein Dekantieren von Glycerin und/oder des wässrigen Teils der Schwerphase, und ein raffinierter Biodiesel-Sammelschritt, gefolgt von einem Schritt der Zugabe eines Antioxidans zu dem raffinierten und gesammelten Biodiesel und/oder einem Schritt des Mischens des raffinierten gesammelten Biodiesels mit einem anderen Kraftstoff, um einen endgültigen 30%igen Biodiesel (B30) zu erhalten.

11. Verfahren nach Anspruch 10, wobei einem wässrigen Teil des Mediums, der das Enzym, das Wasser oder die Base in wässriger Lösung und das während der enzymatischen Umwandlung gebildete Glycerin enthält, Alkohol und verwendete Lipide zur Herstellung von Biodiesel zugesetzt wird, ohne dass das Enzym, das Wasser und/oder die Base und das Glycerin getrennt hinzugefügt werden.

12. Verfahren nach irgendeinem der vorhergehenden Ansprüche, wobei der Alkohol aus der Gruppe bestehend aus Ethanol oder Methanol ausgewählt wird.

13. Verfahren nach irgendeinem der vorhergehenden Ansprüche, wobei Alkohol dem besagten Medium sequenziell 1 bis 4 Mal nach einem Zeitraum zwischen 15 Minuten und 4 Stunden zugesetzt wird.

## Claims

1. Method for obtaining a biodiesel consisting of a mixture of ethyl esters or methyl esters by enzymatic transformation of lipids selected from the group consisting of used vegetable oil, acid vegetable oil and/or used animal fat, wherein said enzymatic transformation comprises an enzymatic transesterification and an enzymatic hydro-esterification, an addition of alcohol, an addition of water or of a base in aqueous solution, and an enzymatic addition of glycerol, **characterized in that** prior to the addition of alcohol, the lipids, the water or the base and glycerol form a homogeneous medium and wherein this medium is brought into contact with one or more enzymes of the triacylglycerol acyl hydrolase type.

2. Method of claim 1, wherein the medium is brought into contact with the enzyme for a duration between 30 minutes and 2 hours.

3. Method of claim 1 or 2, wherein alcohol is added to the molar ratio (alcohol: oil) from (3:1) to (16:1).

4. Method according to any one of the preceding claims, wherein the lipids are subjected, prior to enzymatic transformation into biodiesel, to a cascade decantation step and one or more steps of filtration of the solid particles present in the lipids on filters and to a water removal step, thereby obtaining lipids containing a water content of less than 1% by weight in relation to the weight of the lipids.

5. Method of claim 4, wherein the filters have apertures between 100 µm and 5 µm.

6. Method according to any one of the preceding claims, wherein the lipids consist of used palm oil or of used animal fat that is heated to a temperature of 40 °C or more, preferably 45 °C or more, prior to the filtration step(s).

7. Method according to any one of preceding claims 1 to 5, wherein the lipids are subjected, prior to the enzymatic transformation into biodiesel, to a decantation step at room temperature for used oils and at 45°C or more for animal fats for a duration of between 48 hours and 72 hours.

8. Method according to any one of the preceding claims, comprising a step of recycling the enzyme used.

9. Method according to claim 8 wherein the recycling step of the enzyme used comprises a step for reusing the free enzyme in liquid form, a step of immobilising the enzyme used, a step of collecting the immobilised enzyme used, a step of washing the enzyme with hexane, and a step of drying the collected enzyme.

10. Method according to any one of the preceding claims, comprising after the enzymatic transformation of the used lipids into biodiesel, a refining step of the medium comprising a separation by decantation of a heavy phase which contains glycerol formed during the enzymatic transformation, the water or the base into aqueous solution, and the enzyme, and of an organic phase which contains the biodiesel obtained and the alcohol, a separation of alcohol and biodiesel from the organic phase by evaporation of the alcohol, and a collection by condensation of the evaporated alcohol and/or addition of the recycled alcohol in the method of obtaining biodiesel, a decantation of glycerol and/or of the aqueous portion of the heavy phase, a refined biodiesel collection step, followed by a step of adding an antioxidant, to the refined and collected biodiesel, and/or a step of blending the collected refined biodiesel with another fuel to obtain a final 30% biodiesel (B30).

11. Method of claim 10, wherein an aqueous portion of the medium incorporating the enzyme, water or base in aqueous solution and the glycerol formed during enzymatic transformation, to which the alcohol is added and the used lipids for the obtaining of biodiesel, without separately adding the enzyme, water and/or base and the glycerol.

12. Method according to any one of the preceding claims, wherein the alcohol is selected from the group consisting of ethanol or methanol.

13. Method according to any one of the preceding claims, wherein alcohol is added to said medium sequentially 1 to 4 times, after a duration of between 15 minutes and 4 hours.
